# EUROPEAN PATENT SPECIFICATION

(11) **EP 2 360 480 B1**
(45) Date of publication and mention of the grant of the patent: **22.08.2012**
(21) Application number: 11155153.7
(22) Date of filing: 21.02.2011
(51) Int. Cl.: G01N 33/92

(54) **Method for measuring low density lipoprotein cholesterol**
Verfahren zur Messung von Lipoproteincholesterol mit geringer Dichte
Procédé de mesure de cholestérol de lipoprotéine à faible densité

(30) Priority: 23.02.2010 JP 2010037021
(43) Date of publication of application: 24.08.2011
(73) Proprietor: Fujifilm Corporation, Minato-ku Tokyo 106-0031 (JP)
(72) Inventor: Inomata, Hiroko, Kanagawa 258-8577 (JP); Jumawid, Mariejoy, Kanagawa 258-8577 (JP)
(74) Representative: HOFFMANN EITLE

(56) References cited:
- EP-A1- 1 903 069
- WO-A1-2006/023678
- US-A- 5 795 786

## Description

### Technical Field

The present invention relates to a method for measuring low density lipoprotein cholesterol (LDL-C) in a specimen, wherein a polymer compound having an allylamine or diallylamine unit forms a complex specifically with non LDL (VLDL or HDL).

### Background Art

Lipids present in blood are incorporated into the structure of lipoprotein, except for free fatty acid bound with albumin, and they are present in the form of a chylomicron (CHM), a very low density lipoprotein (VLDL), a low density lipoprotein (LDL), a high density lipoprotein (HDL) and the like. Cholesterol is particularly distributed in VLDL, LDL, and HDL. It has been known that LDL acts to transport cholesterol, and that a high level of LDL promotes arteriosclerosis. It has been known that the measurement of the level of low density lipoprotein cholesterol (LDL-C) in blood becomes an indicator for the development and progression of arteriosclerosis.

Burstein M, et al. (Rapid Method for the Isolation of Lipoproteins from Human Serum by Precipitation with Polyanions, J. Lipid Research 11: 583, 1970) describes that, mainly, lipoproteins other than HDL are specifically agglutinated by using a polyanionic compound. However, it has been difficult for the polyanionic compound described in this document to form a complex specifically with non LDL (VLDL or HDL).

CC Heuck and G Schlierf (Beta-lipoprotein cholesterol quantitation with polycations, Clin Chem 1977 23: 536-540) describes that VLDL and HDL are eliminated by using polyethylenimine and a cationic exchange resin, and that lipoprotein cholesterol is quantified. However, the method described in this document has been problematic in that it has required incubation for 15 minutes or more, and also in that it has required two types of reagents.

### Summary of the Invention

It is an object of the present invention to provide a method capable of measuring low density lipoprotein cholesterol (LDL) in a body fluid with high selectivity.

As a result of intensive studies directed towards achieving the above-mentioned object, the present inventors have discovered a polymer compound having an allylamine or diallylamine unit, which is capable of forming a complex with non LDL. Using the compound of the present invention, it becomes possible that a single compound forms a complex with non LDL (in particular, VLDL and HDL) in a short time. Thereby, it becomes possible to measure ingredients contained in LDL, such as cholesterols, neutral fats and apoproteins. The present invention has been completed based on these findings.

The present invention provides a method for reducing or removing a very low density lipoprotein (VLDL) and a high density lipoprotein (HDL) in a specimen, which comprises treating the specimen containing a very low density lipoprotein (VLDL), a low density lipoprotein (LDL) and a high density lipoprotein (HDL) with a polymer compound having an allylamine or diallylamine unit.

The present invention further provides a method for measuring low density lipoprotein cholesterol (LDL-C) in a body fluid, which comprises treating the body fluid with a polymer compound having an allylamine or diallylamine unit, and measuring the low density lipoprotein cholesterol (LDL-C) using (a) cholesterol esterase and (b) cholesterol oxidase or cholesterol dehydrogenase.

Preferably, a polymer compound having an allylamine or diallylamme unit, which forms a complex with a very low density lipoprotein (VLDL) among lipoproteins, is used.

Preferably, a polymer compound having an allylamine or diallylamine unit, which forms a complex with a high density lipoprotein (HDL) among lipoproteins, is used.

Preferably, the polymer compound having an allylamine or diallylamine unit is any of poly(allylamine), poly(allylamine) hydrochloride, an allylamine hydrochloride copolymer, a diallylamine hydrochloride polymer and a diallylamine hydrochloride copolymer, provided that allylamine and diallylamine may be modified, as desired.

Preferably, the allylamine hydrochloride copolymer and the diallylamine hydrochloride copolymer are a copolymer of allylamine hydrochloride and -SO2-, and a copolymer of diallylamine hydrochloride and -SO2-, respectively.

Preferably, the polymer compound having an allylamine or diallylamine unit is a copolymer of poly(allylamine) or diallylamine hydrochloride and -SO2-.

Preferably, low density lipoprotein cholesterol is measured by allowing peroxidase and a chromogen to act on hydrogen peroxide generated from the low density lipoprotein cholesterol by cholesterol esterase and cholesterol oxidase, so as to carry out a color development reaction.

The present invention further provides a reagent for measuring low density lipoprotein cholesterol (LDL-C), which comprises (a) cholesterol esterase, (b) cholesterol oxidase or cholesterol dehydrogenase, and (c) a polymer compound having an allylamine or diallylamine unit.

The present invention further provides a kit for measuring low density lipoprotein cholesterol (LDL-C), which comprises (a) cholesterol esterase, (b) cholesterol oxidase, (c) peroxidase, (d) a chromogen, and (e) a polymer compound having an allylamine or diallylamine unit.

The present invention further provides a dry analytical element for measuring low density lipoprotein cholesterol (LDL-C), which comprises (a) cholesterol esterase, (b) cholesterol oxidase, (c) peroxidase, (d) a chromogen, and (e) a polymer compound having an allylamine or diallylamine unit.

According to the present invention, low density lipoprotein cholesterol can be measured with high selectivity, using only a single compound in a short time.

### Brief Description of the Drawings

Figure 1 shows agarose gel electrophoresis performed in Example 1 (the results of staining cholesterols).
Figure 2 shows agarose gel electrophoresis performed in Comparative Example 1 (the results of staining cholesterols).
Figure 3 shows the results of the measurement of LDL-C in Example 2.
Figure 4 shows the results of the measurement of LDL-C in Comparative Examples 2.

### Mode for Carrying Out the Invention

Hereinafter, the embodiments of the present invention will be described in detail.

In the present specification, a concentration expressed with % indicates a weight-%-concentration.

The present invention relates to a method for reducing or removing a very low density lipoprotein (VLDL) and a high density lipoprotein (HDL) in a specimen, which comprises treating the specimen containing a very low density lipoprotein (VLDL), a low density lipoprotein (LDL) and a high density lipoprotein (HDL) with a polymer compound having an allylamine or diallylamine unit. Moreover, the present invention relates to a method for measuring low density lipoprotein cholesterol (LDL-C) in a body fluid, which comprises treating the body fluid with a polymer compound having an allylamine or diallylamine unit, and measuring the low density lipoprotein cholesterol (LDL-C) using (a) cholesterol esterase and (b) cholesterol oxidase or cholesterol dehydrogenase.

Blood, serum, plasma, urine or the like can be used as a body fluid. Such blood, serum, plasma or urine may be directly used as a body fluid sample. Otherwise, blood, serum, plasma or urine, which had been pre-treated as appropriate, may also be used.

Examples of a monomer which constitutes the polymer compound having an allylamine or diallylamine unit used in the present invention include allylamine (primary amine), diallylamine (secondary amine), alkyldiallylamine (tertiary amine) and diallyldialkylamine (quaternary amine). The amine may be either a free form, or a salt such as hydrochloride, acetate or sulfate. The amino group may be protected by an alkyl group (for example, a methyl group, an ethyl group, etc.), an alkoxycarbonyl group (for example, a methoxycarbonyl group), or other groups. The polyamine may be a homopolymer of polyamine, or a copolymer formed with polyamine and sulfur dioxide, maleic acid or the like. The polymer compound having an allylamine or diallylamine unit is preferably any of poly(allylamine), poly(allylamine) hydrochloride, an allylamine hydrochloride copolymer, a diallylamine hydrochloride polymer and a diallylamine hydrochloride copolymer, provided that allylamine and diallylamine may be modified, as desired.

It is to be noted that the term "a hydrochloride polymer or copolymer" is used in the present invention, not to mean a product formed by polymerizing monomer hydrochlorides, but to mean that an amine portion of a polymer formed after the polymerization of monomers is a hydrochloride, regardless of production method.

The molecular weight of the polymer compound having an allylamine or diallylamine unit is preferably from 300 or more to 200,000 or less, more preferably from 1,000 or more to 50,000 or less, and further preferably from 1,000 or more to 10,000 or less.

Specific examples of the polymer compound having an allylamine or diallylamine unit used herein include PAA series and PAS series of Nitto Boseki Co., Ltd. The characteristics of the polymer compounds of PAA series and PAS series of Nitto Boseki Co., Ltd., which can be used in the present invention, will be described below. However, the types of the compounds, which can be used in the present invention, are not limited thereto.

**Table 1**

| | | MW |
|---|---|---|
| Poly(allylamine) | PAA-01 | 1,000 |
| | PAA-03 | 3,000 |
| | PAA-05 | 5,000 |
| | PAA-08 | 8,000 |
| | PAA-15C | 15,000 |
| | PAA-25 | 25,000 |
| Diallylamine polymer | PAS-21 | 4,000 |
| Methyl diallylamine hydrochloride polymer | PAS-M-1L | 5,000 |
| | PAS-M-1 | 20,000 |
| Diallylamine-SO2 polymer | PAS-92 | 5,000 |
| Partially methoxycarbonylated allylamine polymer | PAA-U5000 | 15,000 |
| Allylamine hydrochloride-dimethyl allylamine hydrochloride, copolymer | PAA-1112CL | 1,000 |
| Methyl diallylamine hydrorhloride-SO2 copolymer | PAS-2201CL | 4,000 |
| Diallylamine hydrochloride-maleic acid copolymer | PAS-410C | |

**Table 2**

| | | |
|---|---|---|
| polyallylamine hydrochloride PAA-HCI | Polyallylamine PAA | Diallylamine PAS-21 |
| 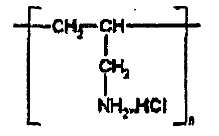 | 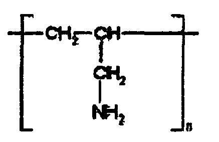 | 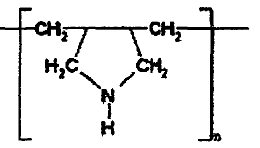 |
| Partially methoxycarbonylated allylamine polymer PAA-U5000 | | Diallylamine-SO2 polymer PAA-92 |
| 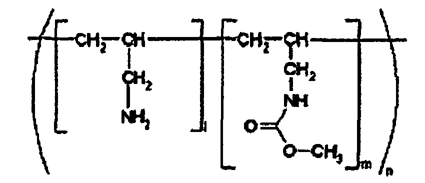 | | 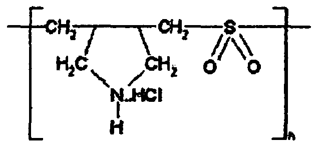 |
| Allylamine hydrochloride-dimethylallylamine Allylamine hydrochloride-dimethylallylamine hydrocholoride copolymer PAA-1112CL | | Methyl diallylamine hydrochloride polymer PA8-M-1 L. PAS-M-1 |
| 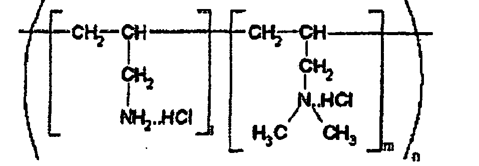 | | 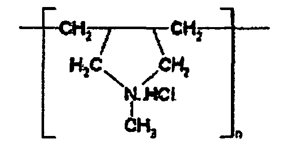 |
| Diallylamine hydrochloride-maleic acid copolymer PAS- 100 | | Methyl diallylamine hydrochloride-sulfur dioxide copolymer PAS-2201CL |
| 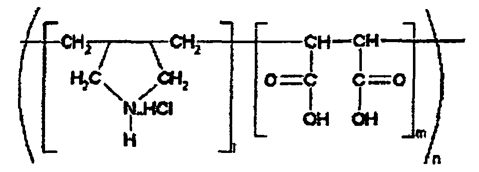 | | 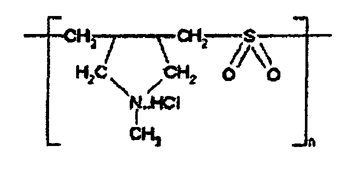 |

| | | |
|---|---|---|
| I and m: a molar ratio n: degree of polymerization | | |

The amount of a polymer compound having an allylamine or diallylamine unit used can be determine as appropriate. When a measurement system is a solution, the final concentration of such a polymer compound having an allylamine or diallylamine unit in a state in which a reagent and a specimen are mixed is 0.001% to 30%, preferably 0.01% to 10%, and more preferably 0.1 % to 5%.

In the case of a dry analytical element in which the measurement system is a dry reagent, the polymer compound having an allylamine or diallylamine unit can be used in an amount of approximately 0.01 to 20 g per m², and more preferably in an amount of approximately 0.1 to 10 g per m².

In order to treat a specimen (a body weight or the like) with a polymer compound having an allylamine or diallylamine unit, the specimen may be mixed with the polymer compound. Then, 1 second to 60 minutes, more preferably 1 second to 30 minutes, and further preferably 5 seconds to 15 minutes after the mixing, the obtained mixture may be subjected to centrifugation or filtration, so as to eliminate non LDL. The specimen can be mixed with the polymer compound having an allylamine or diallylamine unit by a common mixing operation such as upside-down mixing, vortexing or pipetting.

Examples of enzymes used in the method of the present invention include cholesterol esterase, and cholesterol oxidase or cholesterol dehydrogenase. As cholesterol esterase, lipase can also be used. The types of microorganisms, from which such enzymes are derived, are not particularly limited. For example, with regard to cholesterol esterase, *Schizophyllum commune-*derived or Pseudomonas sp.-derived esterase, or esterase derived from other types of microorganisms can be used. With regard to cholesterol oxidase, Pseudomonas sp.-derived, Streptomyces sp.-derived, or other types of microorganisms-derived oxidase can be used. The enzyme used in the present invention may be either an enzyme derived from such microorganisms, or a recombinant enzyme produced by a publicly known method.

An example of cholesterol esterase derived from *Schizophyllum commune* is COE-302 manufactured by Toyobo Co., Ltd. Examples of cholesterol esterase derived from Pseudomonas sp. include COE-311, LPL-312 and LPL-314 manufactured by Toyobo Co., Ltd., and CEN manufactured by Asahi Kasei Corporation. Examples of cholesterol oxidase derived from Pseudomonas sp. include CHO-PEL and CHO-PEWL manufactured by Kikkoman Corporation.

In the method of the present invention, in addition to the aforementioned reagents, reagents for detecting cholesterol, such as an enzyme reagent, a chromogen, and a pH buffer, which are publicly known, can also be used.

More specifically, peroxidase can be used as such enzyme. As chromogen, 4-aminoantipyrine (4-AA), a phenolic or aniline Trinder's reagent that develops color as a result of hydrogen-donating coupling, a leuco dye, or the like can be used. As such Trinder's reagent, an aniline reagent can be preferably used. Examples of such aniline Trinder's reagent used herein include N-ethyl-N-sulfopropyl-3-methoxyaniline (ADPS), N-ethyl-N-sulfopropylaniline (ALPS), N-ethyl-N.sulfopropyl-3-methylaniline (TOPS), N-ethyl-N-(2-hydroxy-3-sulfopropyl)3-methoxyaniline(ADOS), N-ethyl-N-(2-hydroxy-3-sulfopropyl)-3,5-dimethoxyaniline (DAOS), N-(2-hydroxy-3-sulfbpropyl)-3,5-dimetboxyaniline (HDAOS), N-ethyl-N-(2-hydroxy-3-sulfoprapyl)-3,5-dimetlaylaniline (MAOS), and N-ethyl-N-(2-hydroxy-3-sulfopropyl)-3-methoxyaniline (TOOS) (manufactured by Dojindo Laboratories).

Examples of a pH buffer include carbonate, sulfate, phosphate, and a Good's pH buffer described in Biochemistry, 5, pp.467-477,1966, . These pH buffers can be selected with reference to the descriptions of publications, such as Tanpakushitsu Koso no Kiso Jikken Ho (Basic Experimental Methods for Proteins and Enzymes), Takeichi Horio et al., Nankodo Co., Ltd., 1981, and Biochemistry, 5, pp.467-477,1966.

The pH of the aforementioned buffer can be determined depending on the optimal pH of enzyme used. Such pH can be adjusted to preferably pH 5.0 to 8.0, and more preferably pH 6.0 to 7.0.

The measurement method of the present invention in a case in which a measurement system is a solution will be described below. However, the below-mentioned specific embodiment is not intended to limit the scope of the present invention. A reagent solution preferably has a composition which contains the following items (1) to (6).
(1) Cholesterol esterase
(2) Cholesterol oxidase
(3) A polymer compound having an allylamine or diallylamine unit
(4) Peroxidase
(5) Chromogens (4-AA and a Trinder's reagent)
(6) pH buffer

1 to 1,000 µL of, and preferably 100 to 500 µL of a reagent solution prepared by mixing the above-described reagents to each have optimal concentrations is pre-incubated for 1 to 10 minutes, at a constant temperature from approximately 20°C to approximately 45°C, and preferably from approximately 30°C to approximately 40°C. Thereafter, 0.5 to 50 µL of, and preferably 1 to 20 µL of a solution sample is added to the above-described reagent solution. While incubating at a constant temperature, a change over time in wavelength due to the color development of the chromogens is measured. Using the previously prepared calibration curve, the amount of a test substance in a specimen can be obtained in accordance with the principle of colorimetry.

The necessary amounts of enzymes can be determined, as appropriate. For example, all of cholesterol esterase, cholesterol oxidase and peroxidase are used, preferably within the range between 0.2 and 500 U/mL, more preferably within the range between 0.2 and 100 U/mL, and further preferably within the range between 1 and 50 U/mL.

When the measurement system is a solution, one or two or more types of surfactants may be used in combination as necessary. The concentration of such a surfactant is not particularly limited. For example, the surfactant can be used at a concentration of preferably 0.01% to 20%, and more preferably 0.1% to 15%.

The measurement method of the present invention using a dry analytical element, in which the measurement system is a dry reagent, will be described. The dry analytical element can be configured to have at least one adhesion layer and a porous spreading layer on a water-impermeable support.

The porous layer may be made of either a fibrous or non-fibrous material. Since such a porous layer functions as a layer for spreading a liquid sample, it is preferably a layer having an action to measure liquid. The term "an action to measure liquid" is used herein to mean an action to extend, to the lateral orientation of the layer, a liquid sample spotted onto the surface of the layer, at a rate of an almost constant amount per unit area, substantially without uneven distribution of ingredients contained therein. In order to control the spreading area, the spreading rate, and the like, the hydrophilic polymer or surfactant described in JP Patent Publication (Kokai) Nos. 60-222770 A (1985),63-219397 A(1988), and 62-182652 A (1987) can be mixed into the spreading layer. It can also be possible to mix a polycyclic polyglycidol compound as a surfactant into the spreading layer,

The fibrous porous layer is preferably made of polyester fibers, including those described in JP Patent Publication Nos. 55-164356 A (1980), 57-66359 A (1982) and 60-222769 A (1985), as typical examples. The non-fibrous porous layer is preferably made of an organic polymer such as polysulfonic acid.

The adhesion layer is a layer having a function of allowing the above-described water-impermeable support to adhere to the above-described porous layer. Examples of such an adhesion layer that can be used herein include hydrophilic polymers such as gelatin and the derivatives thereof (e.g. phthalate gelatin), cellulose derivatives (e.g. hydroxypropyl cellulose), agarose, acrylamide polymers, methacrylamide polymers, and copolymers of acrylamide or methacrylamide with various types of vinyl monomers.

An aqueous solution containing a hydrophilic polymer is uniformly applied by a publicly known method. Known methods for applying an aqueous solution can be used. In order to apply an aqueous solution containing a hydrophilic polymer, a method may be appropriately selected from among dip coating, extruding coating, doctor coating, hopper coating, curtain coating, and the like, and it may be then used.

It may also be possible to apply the porous layer onto the adhesion layer. However, it is preferable to laminate a cloth or a porous membrane that has previously been supplied as a fabric on the adhesion layer. As a lamination method, as described in JP Patent Publication (Kokai) No. 55-164356 A (1980), the surface of an adhesion layer containing a hydrophilic polymer is uniformly moistened with water, and a cloth or a porous membrane is then placed on the adhesion layer. Thereafter, pressure is lightly and uniformly added thereon, so that the cloth or porous membrane can adhere to the adhesion layer. The thickness of such an adhesion layer is preferably 0.5 to 50 µm, and more preferably 1 to 20 µm.

Preferred materials for a light-permeable support include polyethylene terephthalate, polystyrene, and cellulose ethers such as cellulose triacetate. **In** order to strongly bind a water-absorbing layer as a hydrophilic layer, a detection layer, a substantially non-porous reagent layer and the like to a support, it may be generally possible to establish an undercoating layer on the support, or to perform a hydrophilic treatment thereon. The thickness of the support is not particularly limited. It is preferably 10 to 1,000 µm, and more preferably 300 to 800 µm. In the case of a light-permeable support, the final detection may be carried out either on the support side or the porous layer side. On the other hand, in the case of a light-impermeable support, the final detection is carried out on the porous layer side.

As necessary, a stabilizer, a pH buffer, a crosslinker (a hardener or a curing agent), a surfactant, a polymer, and the like may be used. These agents may be comprised in the adhesion layer or the porous layer.

A preferred layer constitution of a dry analytical element for detecting LDL-C will be described below.

A dry analytical element for detecting LDL-C comprises an adhesion layer coated with a gelatin aqueous solution and a porous layer. The adhesion layer comprises gelatin, peroxidase and a coloring reagent. The adhesion layer is laminated with a cloth or a membrane. Cholesterol esterase and cholesterol oxidase or cholesterol dehydrogenase, a surfactant, and a pH buffer are coated onto the cloth or the membrane.

The reagent composition used for the measurement of cholesterol, which is used in a dry analytical element, and the reagent compositions that bring on an optical change, will be described.

Reagent composition may be contained in a first porous layer. Alternatively, the reagent compositions may be contained in both an adhesion layer and a porous layer. Otherwise, all of, or a majority of the reagent composition may be contained in any of such layers, or such reagent composition may previously be added to any layer(s) other than the adhesion layer and the porous layer.

In a dry analytical element used for LDL-C detection, all enzymes, namely, cholesterol esterase, cholesterol oxidase or cholesterol dehydrogenase, may be each used in an amount of preferably approximately 0.1 to 30 kU per m², and more preferably approximately 0.5 to 15 kU per m².

A surfactant may be used in an amount of approximately 0.2 to 30 g per m², and more preferably approximately 1 to 20 g per m².

The type of peroxidase is not particularly limited. Horseradish peroxidase is preferable. Such peroxidase may be used in an amount of preferably approximately 1 to 200 kU/m², and more preferably approximately 10 to 100 kU/m².

With regard to the above-mentioned chromogen, the combination of the above reagent that couples with 4-ammoantipyrinc (4-AA) to develop color is preferable. Particularly preferably, N-ethyl-N-(2-hydroxy-3-sulfopropyl)-3-methoxyaniline (TOOS) can be used. The amount or such a chromogen used is not particularly limited. For example, 4-AA and a hydrogen-donating coupling agent may be each used in an amount of preferably approximately 0.1 to 10 g/m², and more preferably approximately 0.1 to 5g/m².

As other reagent composition, the dry analytical element used for the detection of LDL-C may further comprise one or two or more types of additives such as a stabilizer, a pH bluffer, a crosslinker (a hardener or a curing agent), a surfactant and a polymer, as necessary. These additives may be contained in the adhesion layer and/or porous layer of the dry analytical element.

The pH of a buffer may be determined depending on the optimal pH of enzyme used. Such pH can be adjusted to preferably pH 5.0 to 8.0, and more preferably pH 6.0 to 7.0.

The dry analytical element can be cut into, for example, small pieces such as a square of approximately 5 mm to approximately 30 mm on a side, or a circle having almost the same size, and it can be then placed in a slide frame described in JP Patent Publication (Kokoku) No. 57-283331 B (1982), JP Utility Model Publication (Kokai) No. 56-142454 U (1981), JP Patent Publication (Kokai) No. 57-63452 A (1982), JP Utility Model Publication (Kokai) No. 58-32350 U (1983), JP Patent Publication (Kohyo) No. 58-501144 A (1983), etc., so that it can be used as a chemical analysis slide. This embodiment is preferable from the viewpoint of production, packaging, transport, storage, measurement procedures, and so on. Depending on the purpose of usage, the dry analytical element may be placed in a long tape form in a cassette or magazine for use. Alternatively, the small piece thereof may be placed in a container with an opening for use, or may be attached to or placed in an opening card for use. Otherwise, the small cut piece may further be used directly.

Upon application of the dry analytical element, an aqueous liquid sample solution (for example, a body fluid sample such as blood or urine) may be spotted in a range of, for example, approximately 2 µL to approximately 30 µL, and preferably 4 µL to 15 µL, onto the porous liquid sample spreading layer in the dry analytical element. Thereafter, the dry analytical element, on which the sample solution has been spotted, may be incubated at a constant temperature ranging from approximately 20°C to approximately 45°C, preferably from approximately 30°C to approximately 40°C, for 1 to 10 minutes. Color development or discoloration in the dry analytical element may be reflex-measured from the light-permeable support side. A calibration curve prepared in advance may be used to determine the amount of the test substance in the specimen according to the principle of colorimetry.

The measurement procedures can be carried out extremely easily using the chemical analyzers described in JP Patent Publication (Kokai) Nos. 60-125543 A (1985), 60-220862 A (1985), 61-294367 A (1986), and 58-161867 A (1983), etc. thereby performing quantitative analysis with high precision. Depending on purposes and necessary precision, the degree of color development may be assessed by visual observation to perform semiquantitative measurement.

The dry analytical element may be stored in a dry state until analysis. Accordingly, the reagents do not have to be prepared before use. Moreover, reagents in a dry state generally have high stability. Therefore, this method is more convenient and rapid than a so-called solution method in which reagent solutions must be prepared before use. Moreover, the method of the present invention is also excellent as an examination method capable of rapid examination with high precision using a trace amount of liquid sample.

The present invention will be more specifically described in the Mowing examples. However, these examples are not intended to limit the scope of the present invention.

### Examples

### (Example 1)

### (1) Preparation of polyamine aqueous solution

Using poly(allylamine) PAA-05 (Nitto Boseki Co., Ltd.) or a diallylamine hydrochloride-sulfur dioxide copolymer PAS-92 (Nitto Boseki Co., Ltd.), a 2% polyamine aqueous solution was prepared with 0.1 M MOPS Buffer (pH 7.0).

### (2) Preparation of lipoprotein solutions

Using a low density lipoprotein LDL (Athens Research & Technology, Inc), a very low density lipoprotein VLDL (Athens Research & Technology, Inc), a high density lipoprotein (Athens Research & Technology, Inc), human serum albumin HSA (Sigma-Aldrich) and human serum globulin HSG (Sigma-Aldrich), the following lipoprotein solutions were prepared.
A mixed solution of 150 mg/dL LDL-C and 60 mg/dL VLDL-C (containing 5% HSA and 2% HSG) A mixed solution of 150 mg/dL LDL-C and 100 mg/dL HDL-C (containing 5% HSA and 2%HSG)

### (3) Detection by agarose gel electrophoresis

100 µL of the 2% polyamine aqueous solution prepared in (1) above was added to 100 µL of the lipoprotein solution prepared in (2) above, and the mixture was then blended by pipetting (the final concentration of polyamine: 1%). Immediately after such blending, the mixture was centrifuged at 3000 rpm for 10 minutes, and a supernatant was then collected.

Using an agarose gel electrophoresis REP system (Helena Laboratories), 1 µL of the supernatant was electrophoresed on an REP LIPO-30 plate (Helena Laboratories). Cholesterols and neutral fats were stained with Chol/Trig Combo CWTG and a lipoprotein remaining in the supernatant was then detected.

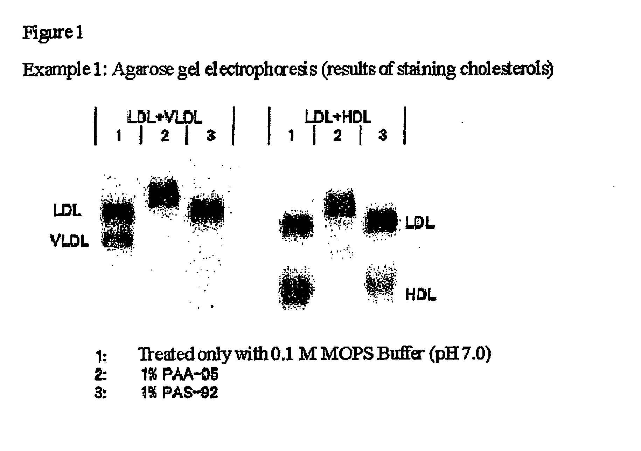

Figure 1 shows the results obtained in the case of using an LDL-VLDL mixed solution as a specimen and the results obtained in the case of using an LDL-HDL mixed solution as a specimen. In the left view of Figure 1, it is found that when compared with only 0.1 M MOPS Buffer (lane 1), a band of VLDL has disappeared in both PAA-05 (lane 2) and PAS-92 (lane 3). Moreover, in the right view of Figure 1, it is found that a band of HDL has disappeared in PAA-05 (lane 2), and that the concentration of such an HDL band has been decreased to approximately 1/3 of the initial concentration in PAS-92 (lane 3).

From these results, it was confirmed that, in particular VLDL, and also HDL, from among non LDLs, can be removed by using the polyamine aqueous solution of the present invention.

### (Comparative Example 1)

### (1) Preparation of polyethylenimine aqueous solution

Using polyethylenimine 1800 (PEI) (Wako Pure Chemical Industries, Ltd.), a 2% polyethylenimine aqueous solution was prepared with 0.1 M MOPS Buffer (pH 7.0).

### (2) Preparation of lipoprotein solutions

Using a low density lipoprotein LDL (Athens Research & Technology, Inc), a very low density lipoprotein VLDL (Athens Research & Technology, Inc), a high density lipoprotein (Athens Research & Technology, Inc), human serum albumin HSA (Sigma-Aldrich) and human serum globulin HSG (Sigma-Aldrich), the following lipoprotein solutions were prepared.
60 mg/dL VLDL-C mixed solution (containing 5% HSA and 2% HSG)
A mixed solution of 150 mg/dL LDL-C and 60 mg/dL VLDL-C (containing 5% HSA and 2% HSG) 100 mg/dL HDL-C mixed solution (containing 5% HSA and 2% HSG)
A mixed solution of 150 mg/dL LDL-C and 100 mg/dL HDL-C (containing 5% HSA and 2%HSG) (3) Detection by agarose gel electrophoresis

100 µL of the 2% polyethylenimine aqueous solution prepared in (1) above was added to 100 µL of the lipoprotein solution prepared in (2) above, and the mixture was then blended by pipetting (the final concentration of polyethylenimine: 1%). Immediately after such blending, the mixture was centrifuged at 3000 rpm for 10 minutes, and a supernatant was then collected.

Using an agarose gel electrophoresis REP system (Helena Laboratories), 1 µL of the supernatant was electrophoresed on an REP LIPO-30 plate (Helena Laboratories). Cholesterols and neutral fats were stained with Chol/Trig Combo CH/TG, and a lipoprotein remaining in the supernatant was then detected.

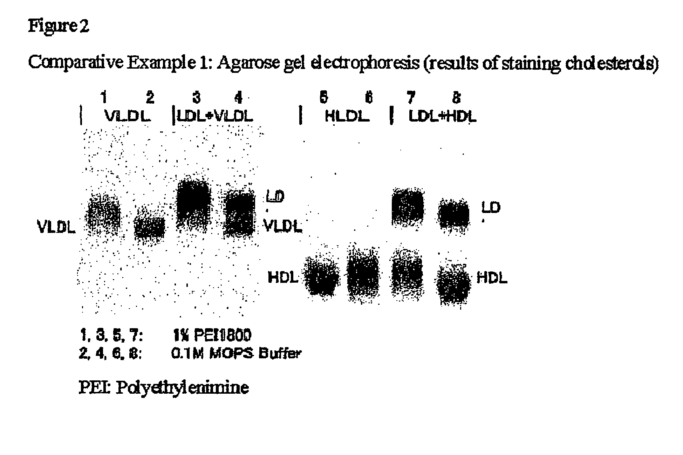

Figure 2 shows the results obtained in the case of using a VLDL solution and an LDL-VLDL mixed solution as specimens, and the results obtained in the case of using an HDL solution and an LDL-HDL mixed solution as specimens. In the left view of Figure 2, it is found that when compared with only 0.1 M MOPS Buffer, a band of VLDL is shifted when it has been treated with PEI, and it remains in a large amount. In addition, in the right view of Figure 2 as well, a band of HDL has not disappeared, and it remains in a large amount. From these results, it is found that VLDL and HDL cannot be removed by the use of polyethylenimine.

### (Example 2)

### (1) Preparation of lipoprotein solutions

Using a low density lipoprotein LDL (Athens Research & Technology, Inc), a very low density lipoprotein VLDL (Athens Research & Technology, Inc), a high density lipoprotein (Athens Research & Technology, Inc), human serum albumin HSA (Sigma-Aldrich) and human scrum globulin HSG (Sigma-Aldrich), the following lipoprotein solutions were prepared.
50 mg/dL LDL-C (containing 5% HSA and 2% HSG)
75 mg/dL LDL-C (containing 5% HSA and 2% HSG)
150 mg/dL LDL-C (containing 5% HSA and 2% HSG)
250 mg/dL LDL-C (containing 5% HSA and 2% HSG)
A mixed solution of 75 mg/dL LDL-C and 30 mg/dL VLDL-C (containing 5% HSA and 2% HSG)
A mixed solution of 75 mg/dL LDL-C and 60 mg/dL VLDL-C (containing 5% HSA and 2% HSG)
A mixed solution of 150 mg/dL LDL-C and 50 mg/dL HDL-C (containing 5% HSA and 2% HSG)
A mixed solution of 150 mg/dL LDL-C and 100 mg/dL HDL-C (containing 5% HSA and 2% HSG)

### (2) Dry analytical element for measuring LDL-C

A gelatin aqueous solution containing 4-aminoantipynne, TOOS (manufactured by Dojindo Laboratories) and peroxidase (Toyobo Co., Ltd.) was coated onto a 180-µm polyethylene terephthalate colorless transparent smooth film that had been undercoated with gelatin, resulting in the coating thickness of 14 µm after drying, and it was then dried. Subsequently, water was supplied to the entire surface of the above-described film at a supply amount of approximately 30 g/m², so as to cause the film get wet. A tricot knit fabric prepared by knitting an about 50-denier polyester spun yarn at 36 gauges was lightly pressed on the resultant film, so that it was laminated thereon, and it was then dried. Thereafter, an aqueous solution having the following composition was coated onto the above-described fabric, and it was then dried.

| | |
|---|---|
| MOPS (pH 7.0) | 1.67 glm² |
| Polyglyceryl distyl phenyl ether | 2.98 glm² |
| Pluronic L121 | 14.90 g/m² |
| Cholesterol esterase (lipoprotein lipase, Toyobo Co., Ltd.) | 3.21 kU/m² |
| Cholesterol oxidase (recombinant *E. Coli,* Kikkoman Corp.) | 4.52 kU/m² |
| PVP K90 | 15.00 g/m² |
| PAS-92 | 1.03 g/m² |

### (3) Evaluation of LDL-C measurement

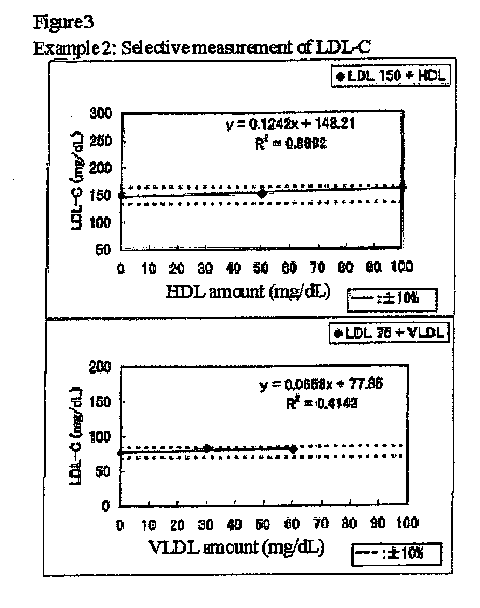

10 µL of the lipoprotein solution prepared in (1) above was spotted onto the slide produced in (2) above. It was then reacted at 37°C for 6 minutes, and OD at 540 nm was measured 6 minutes alter the initiation of the reaction. A calibration curve was produced with a specimen of only LDL, and the cholesterol concentrations of other samples (LDL + non LDL) were then calculated based on the calibration curve. As shown in Figure 3, it was confirmed that non LDL was not measured so much together with LDL-C in the case of mixed specimens consisting of non LDL (HDL or VLDL) and LDL.

### (Comparative Example 2)

### (1) Preparation of lipoprotein solutions

Using a low density lipoprotein LDL (Athens Research & Technology, Inc), a very low density lipoprotein VLDL (Athens Research & Technology, Inc), a high density lipoprotein (Athens Research & Technology, Inc), human serum albumin HSA (Sigma-Aldrich) and human serum globulin HSG (Sigma-Aldrich), the following lipoprotein solutions were prepared.
50 mg/dL LDL-C (containing 5% HSA and 2% HSG)
75 mg/dL LDL-C (containing 5% HSA and 2% HSG)
150 mg/dL LDL-C (containing 5% HSA and 2% HSCB)
250 mg/dL LDL-C (containing 5% HSA and 2% HSG)
A mixed solution of 75 mg/dL LDL-C and 30 mg/dL VLDL-C (containing 5% HSA and 2% HSG)
A mixed solution of 75 mg/dL LDL-C and 60 mg/dL VLDL-C (containing 5% HSA and 2% HSG)
A mixed solution of 150 mg/dL LDL-C and 50 mg/dL HDL-C (containing 5% HSAand 2% HSG)
A mixed solution of 150 mg/dL LDL-C and 100 mg/dL HDL-C (containing 5% HSA and 2% HSG)

### (2) Dry analytical element for measuring LDL-C

A gelatin aqueous solution containing 4-aminoantipyrine, TOOTS (manufactured by Dojindo Laboratories) and peroxidase (Toyobo Co., Ltd.) was coated onto a 180-µm polyethylene terephthalate colorless transparent smooth film that had been undercoated with gelatin, resulting in a coating thickness of 14 µm after drying, and it was then dried. Subsequently, water was supplied to the entire surface of the above-described film at a supply amount of approximately 30 g/m², so as to cause the film get wet. A tricot knit fabric prepared by knitting an about 50-denier polyester spun yarn at 36 gauges was lightly pressed on the resultant film, so that it was laminated thereon, and it was then dried. Thereafter, an aqueous solution having the following composition was coated onto the above-described fabric, and it was then dried.

| | |
|---|---|
| MOPS (pH 7.0) | 1.67 g/m² |
| Polyglyceryl distyl phenyl ether | 2.98 g/m² |
| Pluronic L121 | 14.90 g/m² |
| Cholesterol esterase (lipoprotein lipase, Toyobo Co., Ltd.) | 3.21 kU/m² |
| Cholesterol oxidase (recombinant *E. Coli,* Kikkoman Corp.) | 4.52 kUfm² |

### (3) Evaluation of LDL-C measurement

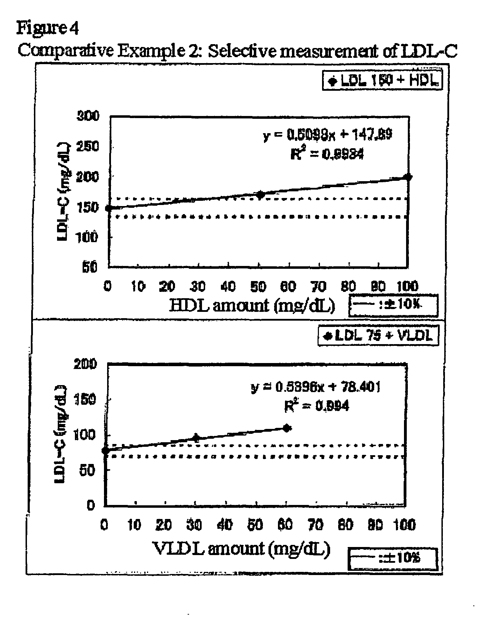

10 µL of the lipoprotein solution prepared in (1) above was spotted onto the slide produced in (2) above. It was then reacted at 37°C for 6 minutes, and OD at 540 nm was measured 6 minutes after the initiation of the reaction. A calibration curve was produced with a specimen of only LDL, and the cholesterol concentrations of other samples (LDL + non LDh) were then calculated based on the calibration curve. As shown in Figure 4, it was confirmed that a large amount of non LDL was measured together with LDL-C (LDL concentration: 110% or more) in the case of mixed specimens consisting of non LDL (HDL or VLDL) and LDL.

## Claims

1. A method for reducing or removing a very low density lipoprotein (VLDL) and a high density lipoprotein (HDL) in a specimen, which comprises treating the specimen containing a very low density lipoprotein (VLDL), a low density lipoprotein (LDL) and a high density lipoprotein (HDL) with a polymer compound having an allylamine or diallylamine unit.

2. A method for measuring low density lipoprotein cholesterol (LDL-C) in a sample of a body fluid, which comprises treating the body fluid with a polymer compound having an allylamine or diallylamine unit, and measuring the low density lipoprotein cholesterol (LDL-C) using (a) cholesterol esterase and (b) cholesterol oxidase or cholesterol dehydrogenase.

3. The method according to claim 2, wherein a polymer compound having an allylamine or diallylamine unit, which forms a complex with a very low density lipoprotein (VLDL) among lipoproteins, is used.

4. The method according to claim 2, wherein a polymer compound having an allylamine or diallylamine unit, which forms a complex with a high density lipoprotein (HDL) among lipoproteins, is used.

5. The method according to claim 2, wherein the polymer compound having an allylamine or diallylamine unit is any of poly(allylamine), poly(allylamine) hydrochloride, an allylamine hydrochloride copolymer, a diallylamine hydrochloride polymer and a diallylamine hydrochloride copolymer, provided that allylamine and diallylamine may be modified, as desired.

6. The method according to claim 5, the allylamine hydrochloride copolymer and the diallylamine hydrochloride copolymer are a copolymer of allylamine hydrochloride and -SO2-, and a copolymer of diallylamine hydrochloride and -SO2-, respectively

7. The method according to claim 4, wherein the polymer compound having an allylamine or diallylamine unit is a copolymer of poly(allylamine) or diallylamine hydrochloride and -SO2-.

8. The method according to claim 2, wherein low density lipoprotein cholesterol is measured by allowing peroxidase and a chromogen to act on hydrogen peroxide generated from the low density lipoprotein cholesterol by cholesterol esterase and cholesterol oxidase, so as to carry out a color development reaction.

9. A reagent for measuring low density lipoprotein cholesterol (LDL-C), which comprises (a) cholesterol esterase, (b) cholesterol oxidase or cholesterol dehydrogenase, and (c) a polymer compound having an allylamine or diallylamine unit

10. A kit for measuring low density lipoprotein cholesterol (LDL-C), which comprises (a) cholesterol esterase, (b) cholesterol oxidase, (c) peroxidase, (d) a chromogen, and (e) a polymer compound having an allylamine or diallylamine unit.

11. A dry analytical element for measuring low density lipoprotein cholesterol (LDL-C), which comprises (a) cholesterol esterase, (b) cholesterol oxidase, (c) peroxidase, (d) a chromogen, and (e) a polymer compound having an allylamine or diallylamine unit

## Patentansprüche

1. Verfahren zur Reduzierung oder Entfernung eines Lipoproteins sehr niedriger Dichte (VLDL) und eines Lipoproteins hoher Dichte (HDL) in einer Probe, umfassend die Behandlung der Probe, umfassend ein Lipoprotein sehr niedriger Dichte (VLDL), ein Lipoprotein niedriger Dichte (LDL) und ein Lipoprotein hoher Dichte (HDL) mit einer Polymerverbindung mit einer Allylamin- oder Diallylamineinheit.

2. Verfahren zur Messung von niedrigdichtem Lipoprotein-Cholesterin (LDL-C) in einer Probe eines Körperfluids, umfassend die Behandlung des Körperfluids mit einer Polymerverbindung mit einer Allylamin- oder Diallylamineinheit und Messen des niedrigdichten Lipoprotein-Cholesterins (LDL-C) unter Verwendung (a) von Cholesterolesterase und (b) Cholesteroloxidase oder Cholesteroldehydrogenase.

3. Verfahren nach Anspruch 2, worin eine Polymerverbindung mit einer Allylamin- oder Diallylamineinheit, die einen Komplex mit Lipoprotein sehr niedriger Dichte (VLDL) unter Lipoproteinen bildet, verwendet wird.

4. Verfahren nach Anspruch 2, worin eine Polymerverbindung mit einer Allylamin- oder Diallylamineinheit, die einen Komplex mit Lipoprotein hoher Dichte (HDL) unter Lipoproteinen bildet, verwendet wird.

5. Verfahren nach Anspruch 2, worin die Polymerverbindung mit einer Allylamin- oder Diallylamineinheit eine Verbindung von Poly(allylamin), Poly(allylamin)hydrochlorid, einem Allylaminhydrochlorid-Copolymer, einem Diallylaminhydrochlorid-Polymer und einem Diallylaminhydrochlorid-Copolymer ist, vorausgesetzt, dass Allylamin und Diallylamin nach Wunsch modifiziert werden können.

6. Verfahren nach Anspruch 5, worin das
Allylaminhydrochlorid-Copolymer und das Diallylaminhydrochlorid-Copolymer ein Copolymer aus Allylaminhydrochlorid und -SO₂- beziehungsweise ein Copolymer aus Diallylaminhydrochlorid und -SO₂- sind.

7. Verfahren nach Anspruch 4, worin die Polymerverbindung mit einer Allylamin- oder Diallyleinheit ein Polymer aus Poly(allylamin) oder Diallylhydrochlorid und -SO₂- ist.

8. Verfahren nach Anspruch 2, worin niedrigdichtes Lipoprotein-Cholesterin gemessen wird, indem ermöglicht wird, dass Peroxidase und ein Chromogen auf Wasserstoffperoxid agiert, das von dem niedrigdichten Lipoprotein-Cholesterin durch Cholesterolesterase und Cholesteroloxidase erzeugt ist, zur Durchführung einer Farbentwicklungsreaktion.

9. Reagens zum Messen von niedrigdichtem Lipoprotein-Cholesterin (LDL-C), umfassend (a) Cholesterolesterase, (b) Cholesteroloxidase oder Cholesteroldehydrogenase und (c) eine Polymerverbindung mit einer Allylamin- oder Diallylamineinheit.

10. Kit zum Messen von niedrigdichtem Lipoprotein-Cholesterin (LDL-C), umfassend (a) Cholesterolesterase, (b) Cholesteroloxidase, (c) Peroxidase, (d) ein Chromogen und (e) eine Polymerverbindung mit einer Allylamin- oder Diallylamineinheit.

11. Trockenes analytisches Element zum Messen von niedrigdichtem Lipoprotein-Cholesterin (LDL-C), umfassend (a) Cholesterolesterase, (b) Cholesteroloxidase, (c) Peroxidase, (d) ein Chromogen und (e) eine Polymerverbindung mit einer Allylamin- oder Diallylamineinheit.

## Revendications

1. Procédé pour réduire ou éliminer une lipoprotéine très basse densité (VLDL) et une lipoprotéine haute densité (HDL) dans un échantillon, qui comprend le traitement de l'échantillon contenant une lipoprotéine très basse densité (VLDL), une lipoprotéine basse densité (LDL) et une lipoprotéine haute densité (HDL) avec un composé polymère ayant un motif allylamine ou diallylamine.

2. Procédé pour mesurer le cholestérol de lipoprotéine basse densité (LDL-C) dans un échantillon d'un fluide corporel, qui comprend le traitement du fluide corporel avec un composé polymère ayant un motif allylamine ou diallylamine, et la mesure du cholestérol de lipoprotéine basse densité (LDL-C) par utilisation (a) d'une cholestérol estérase et (b) d'une cholestérol oxydase ou cholestérol déshydrogénase.

3. Procédé selon la revendication 2, dans lequel est utilisé un composé polymère ayant un motif allylamine ou diallylamine, qui forme un complexe avec, parmi les lipoprotéines, une lipoprotéine très basse densité (VLDL).

4. Procédé selon la revendication 2, dans lequel est utilisé un composé polymère ayant un motif allylamine ou diallylamine, qui forme un complexe avec, parmi les lipoprotéines, une lipoprotéine haute densité (HDL).

5. Procédé selon la revendication 2, dans lequel le composé polymère ayant un motif allylamine ou diallylamine est l'un quelconque parmi la poly(allylamine), le poly(chlorhydrate d'allylamine), un copolymère de chlorhydrate d'allylamine, un polymère de chlorhydrate de diallylamine et un copolymère de chlorhydrate de diallylamine, sous réserve que l'allylamine et la diallylamine puissent être modifiées si cela est souhaité.

6. Procédé selon la revendication 5, dans lequel le copolymère de chlorhydrate d'allylamine et le copolymère de chlorhydrate de diallylamine sont respectivement un copolymère de chlorhydrate d'allylamine et de -SO₂-, et un copolymère de chlorhydrate de diallylamine et de -SO₂-.

7. Procédé selon la revendication 4, dans lequel le composé polymère ayant un motif allylamine ou diallylamine est un copolymère de poly(allylamine) ou de chlorhydrate de diallylamine et de -SO₂-.

8. Procédé selon la revendication 2, dans lequel le cholestérol de lipoprotéine basse densité est mesuré par l'opération consistant à laisser une peroxydase et un chromogène agir sur le peroxyde d'hydrogène généré à partir du cholestérol lipoprotéine basse densité par une cholestérol estérase et une cholestérol oxydase, de façon que se déroule une réaction de développement de couleur.

9. Réactif pour mesurer le cholestérol de lipoprotéine basse densité (LDL-C), qui comprend (a) une cholestérol estérase, (b) une cholestérol oxydase ou cholestérol déshydrogénase, et (c) un composé polymère ayant un motif allylamine ou diallylamine.

10. Trousse pour mesurer le cholestérol de lipoprotéine basse densité (LDL-C), qui comprend (a) une cholestérol estérase, (b) une cholestérol oxydase, (c) une peroxydase, (d) un chromogène, et (e) un composé polymère ayant un motif allylamine ou diallylamine.

11. Elément analytique sec pour mesurer le cholestérol de lipoprotéine basse densité (LDL-C), qui comprend (a) une cholestérol estérase, (b) une cholestérol oxydase, (c) une peroxydase, (d) un chromogène, et (e) un composé polymère ayant un motif allylamine ou diallylamine.
